# EUROPEAN PATENT SPECIFICATION

(11) **EP 2 913 063 B1**
(45) Date of publication and mention of the grant of the patent: **11.09.2019**
(21) Application number: 13848825.9
(22) Date of filing: 23.10.2013
(51) Int. Cl.: A61K 45/00, A61K 38/04, A61P 21/02, C07K 14/46

(54) **THERAPEUTIC AGENT FOR USE IN TREATING AMYOTROPHIC LATERAL SCLEROSIS**
THERAPEUTIKUM ZUR VERWENDUNG BEI DER BEHANDLUNG VON AMYOTROPHISCHE LATERALSKLEROSE
AGENT THÉRAPEUTIQUE POUR SON UTILISATION DANS LE TRAITEMENT DE LA SCLÉROSE LATÉRALE AMYOTROPHIQUE

(30) Priority: 24.10.2012 JP 2012234300
(43) Date of publication of application: 02.09.2015
(73) Proprietor: Daiichi Sankyo Company, Limited, Tokyo 103-8426 (JP)
(72) Inventor: MATSUO, Tsuyoshi, Kobe-shi Hyogo 650-0047 (JP); MURAYAMA, Norihito, Kobe-shi Hyogo 650-0047 (JP); FURUYA, Mayumi, Kobe-shi Hyogo 650-0047 (JP)
(74) Representative: Arends, William Gerrit
(86) International application number: PCT/JP2013/078743
(87) International publication number: WO 2014/065341

(56) References cited:
- EP-A1- 1 258 250
- WO-A2-2006/079077
- DIANA GARCÍA DEL BARCO ET AL: "Therapeutic Effect of the Combined Use of Growth Hormone Releasing Peptide-6 and Epidermal Growth Factor in an Axonopathy Model", NEUROTOXICITY RESEARCH, vol. 19, no. 1, 19 February 2010 (2010-02-19), pages 195-209, XP055277438, CH ISSN: 1029-8428, DOI: 10.1007/s12640-010-9160-8
- B. HOLST ET AL: "Overlapping Binding Site for the Endogenous Agonist, Small-Molecule Agonists, and Ago-allosteric Modulators on the Ghrelin Receptor", MOLECULAR PHARMACOLOGY, vol. 75, no. 1, 1 January 2009 (2009-01-01), pages 44-59, XP055277365, US ISSN: 0026-895X, DOI: 10.1124/mol.108.049189
- LEE S ET AL.: 'Ghrelin protects spinal cord motoneurons against chronic glutamate excitotoxicity by inhibiting microglial activation' KOREAN J PHYSIOL PHARMACOL vol. 16, no. 1, February 2012, pages 43 - 8, XP055252677
- LIM E ET AL.: 'Ghrelin protects spinal cord motoneurons against chronic glutamate-induced excitotoxicity via ERK1/2 and phosphatidylinositol-3-kinase/Akt/glycogen synthase kinase-3p pathways' EXP NEUROL vol. 230, no. 1, 2011, pages 114 - 22, XP055252678
- ISAO NAGANO MODERN PHYSICIAN vol. 22, no. 5, 2002, pages 579 - 584, XP008179503

### SEQUENCE LISTING

<110> Daiichi Sankyo Company, Limited
<120> Therapeutic agent for amyotrophic lateral sclerosis
<130> PN816417EP
<140> EP13848825.9
   <141> 2013-10-23
<150>\201 JP 2012-234300
   <151>\201 2012-10-24
<160> 2
<210> 1
   <211> 28
   <212> PRT
   <213> Homo sapiens
<220>
   <221> PEPTIDE
   <222> (1)..(28)
   <223> Amino acid sequence for human endogenous peptides of growth hormo ne secretagogue
<400> 1
   Gly Ser Ser Phe Leu Ser Pro Glu His Gln Arg Val Gln Gln Arg Lys
   1               5                   10                  15
   Glu Ser Lys Lys Pro Pro Ala Lys Leu Gln Pro Arg
               20                  25
<210> 2
   <211> 27
   <212> PRT
   <213> Homo sapiens
<220>
   <221> PEPTIDE
   <222> (1)..(27)
   <223> Amino acid sequence for human endogenous peptides (27 amino acids ) of growth hormone secretagogue
<400> 2
   Gly Ser Ser Phe Leu Ser Pro Glu His Gln Arg Val Gln Arg Lys Glu
   1               5                   10                  15
   Ser Lys Lys Pro Pro Ala Lys Leu Gln Pro Arg
               20                  25

## Description

### Technical Field

The present invention relates to a therapeutic agent for amyotrophic lateral sclerosis comprising a growth hormone secretagogue receptor agonist as an active ingredient, wherein the growth hormone secretagogue receptor agonist is ghrelin, GHRP-6, or anamorelin.

### Background Art

Amyotrophic lateral sclerosis (hereinafter, referred to as ALS), the most common motor neuron disease of adults, is a neurodegenerative disease involving selective and systemic death of upper and lower motor neurons. As a result of the motor neuron death, muscle wasting and muscle weakness of the upper and lower limbs occurs, while in most cases the progress of the disease also involves bulbar palsy symptoms such as difficulties in speech or swallowing, and respiratory muscle paralysis. ALS mostly strikes in middle age or later. This disease is so severe that many patients die of respiratory failure within 2 to 3 years after the onset unless respiratory management using respirators is conducted. High-order functions such as intelligence and sensation are maintained, in spite of the systemic wasting and weakness of muscles including respiratory muscles (Non Patent Literature 1).

Cu/Zn superoxide dismutase (SOD1) gene is considered as one of the causative gene of ALS. In transgenic mice or rats harboring mutant SOD1 genes isolated from ALS patients, it has been found that motor neurons selectively die after maturation and skeletal muscle wasting or muscle weakness proceeds, resulting in death, as in the pathology of human ALS. These transgenic mice or rats harboring mutant SOD1 genes have been established as ALS animal models and routinely used in the pathological analysis of ALS or search for therapeutic agents for this disease. Particularly, research using transgenic mice expressing mutant proteins derived from human SOD1 by the substitution of Gly (G) at the 93-position with Ala (A), i.e., transgenic mice harboring the mutant SOD1 gene (G93A) (hereinafter, referred to as SOD1^{G93A} mice) has been most advanced, and evaluation using these animals is recommended by the European ALS/MND group (Non Patent Literature 2).

The only therapeutic agent for ALS approved around the world is currently riluzole. This drug antagonizes glutamate toxicity, which is reportedly one of the factors responsible for the onset of ALS. The drug, however, produces a life-prolonging effect of only few months and is thus only effective to a limited extent (Non Patent Literature 1).

Ghrelin is a peptide hormone discovered as an endogenous ligand of a growth hormone secretagogue receptor (hereinafter, referred to as GHS-R) (Non Patent Literature 3). Ghrelin promotes growth hormone (hereinafter, referred to as GH) secretion in humans and animals (Non Patent Literature 4, etc.).

GH is known to promote insulin-like growth factor-1 (hereinafter, referred to as IGF-1) production in the liver and skeletal muscles, while IGF-1 is known as a trophic factor for motor neurons. The injection of a recombinant AAV4 virus vector containing the IGF-1 gene into the lateral ventricles of SOD1^{G93A} mice significantly prolonged their survival periods and significantly suppressed reduction in motor function and muscle strength (Patent Literature 1). Although this report showed that such effects were brought about by the forced expression of IGF-1 in the brain, the overexpression of human IGF-1 in the skeletal muscles of SOD1^{G93A} mice had no influence on motor neuron death or survival periods (Non Patent Literature 5). When GH or IGF-1 was administered to ALS patients in clinical trials, the GH therapy was ineffective (Non Patent Literature 6) and IGF-1 exhibited no efficacy on any of the muscle strength, functional outcomes, and survival periods of ALS patients (Non Patent Literature 7). Thus, the activation of the peripheral GH or IGF-1 system is not effective for ALS. Ghrelin also promotes GH secretion from the hypophysis and increases GH concentrations in blood (e.g., Non Patent Literature 3 and 4), but is not known to have the effect of increasing IGF-1 in the brain or the spinal cord. It is therefore uncertain whether ghrelin is effective for the pathology of ALS.

Also, ghrelin is the only known peripheral orexigenic peptide that reportedly increases food intakes in humans and animals (e.g., Non Patent Literature 4). ALS is a disease involving wasting of the skeletal muscles and loss of muscle strength caused by motor neuron death, resulting in death. The administration of ghrelin may increase food intake and thereby maintain body weights or skeletal muscle masses. The relationship between such effects of ghrelin and the suppression of motor neuron death is, however, unknown. It is therefore uncertain whether ghrelin is effective in relation to the pathology of ALS.

Among ALS patients, patients who have a high blood LDL/HDL ratio and manifest hyperlipidemia have been reported to exhibit a longer survival period by at least 12 months than other ALS patients, indicating that hyperlipidemia is a prognostic factor for the survival of ALS patients (Non Patent Literature 8). Likewise, ALS patients having a high blood cholesterol or triglyceride concentration have been reported to have good prognosis (Non Patent Literature 9).

According to one report, the administration of ghrelin to mice had no influence on their blood triglyceride concentrations, but increased the blood total cholesterol concentrations (Non Patent Literature 10). According to another report, ghrelin is useful in the treatment of hyperlipidemia (Patent Literature 2).

Also, the 3-weeks of repeated administration of ghrelin to patients with chronic respiratory inflammation improved their body weights or nutritional status, but had no influence on blood cholesterol concentrations (Non Patent Literature 11).

As mentioned above, definite findings have not yet been obtained as to the influence of ghrelin on blood cholesterol or triglyceride.

When cell death was induced by the elevation of intracellular calcium (Ca) levels through the ionomycin treatment of fetal rat hippocampal neurons, GHS-R agonist compounds rat ghrelin, pralmorelin (growth hormone releasing peptide-2 (hereinafter, referred to as GHRP-2)), hexarelin, and MK-0677 reportedly exhibited a cell death suppressive effect. In this report, ALS is named as a non-ischemic neurodegenerative disease for which the compound group is effective (Patent Literature 3). ALS is an adult-onset disease involving the selective death of motor neurons in which hippocampal cells are not damaged. Accordingly, even if GHRP-2 or ghrelin suppressed *in vitro* the fetal rat hippocampal neuron death induced by the elevation of Ca concentrations, it is uncertain whether these compounds are effective for ALS.

Ghrelin has been reported to suppress motor neuron death induced by glutamate in an *in vitro* rat organotypic spinal cord culture system (Non Patent Literature 12 and 13). Unfortunately, the *in vitro* organotypic spinal cord culture system may not sufficiently reflect the pathology of a complicated disease such as ALS.

Pralmorelin (GHRP-2) has been reported to increase the proportion of rat adrenal pheochromocytoma-derived PC-12 cells having dendrites and axons 1.5 times larger than the size of the cells, by approximately 1.2 times compared with a control group (Patent Literature 4). Since the cells are not motor neurons, it cannot be judged whether or not GHRP-2 is useful for ALS.

According to a report on mouse model of proximal axonopathy induced by the administration of 1,2-diacetylbenzene, the simultaneous administration of GHS-R agonist growth hormone releasing peptide-6 (hereinafter, referred to as GHRP-6) and cell growth factor EGF behaviorally improved the ability of the mice to walk and also significantly improved the action potentials of the skeletal muscles, though the administration of each compound alone merely accelerated the recovery of altered gait patterns (Non Patent Literature 14). Since the animal models used in this experiment had distinct clinical pathology compared with ALS, it cannot be judged whether GHRP-6 is useful or non-useful for ALS.

Further reports disclose that a ghrelin receptor GHS-R antagonist is useful in the treatment of ALS (Patent Literature 5 and 6). Thus, GHS-R agonists including ghrelin cannot be presumed to be useful in the treatment of ALS.

### Citation List

### Patent Literature

Patent Literature 1: WO2007/146046
Patent Literature 2: Japanese Patent Laid-Open No. 2008-127377
Patent Literature 3: WO01/047558
Patent Literature 4: Japanese Patent Laid-Open No. 2005-239712
Patent Literature 5: US7829589
Patent Literature 6: US2012/0080747

### Non Patent Literature

Non Patent Literature 1: Expert Opinion on Emerging Drugs (2011), vol. 16 (No. 2), p. 537-558
Non Patent Literature 2: Amyotrophic Lateral Sclerosis (2010), vol. 11, p. 38-45
Non Patent Literature 3: Nature (1999), vol. 402, p. 656-660
Non Patent Literature 4: Physiological Reviews (2005), vol. 85, p. 495-522
Non Patent Literature 5: Experimental Neurology (2007), vol. 207, p. 52-63
Non Patent Literature 6: Muscle & Nerve (1993), vol. 16 (No. 6), p. 624-633
Non Patent Literature 7: Neurology (2008), vol. 71, p. 1770-1775
Non Patent Literature 8: Neurology (2008), vol. 70, p. 1004-1009
Non Patent Literature 9: Journal of Neurology (2011), vol. 258 (No. 4), p. 613-617
Non Patent Literature 10: Nature Neuroscience (2010), vol. 13 (No. 7), p. 877-883
Non Patent Literature 11: Pulmonary Pharmacology & Therapeutics (2008), vol. 21 (No. 5), p. 774-779
Non Patent Literature 12: Experimental Neurology (2011), vol. 230, p. 114-122
Non Patent Literature 13: Korean Journal of Physiology and Pharmacology (2012), vol. 16 (No. 1), p. 43-48
Non Patent Literature 14: Neurotoxicity Research (2011), vol. 19, p. 195-209

### Summary of Invention

### Technical Problem

Riluzole, an existing therapeutic agent for ALS, is clinically effective to a limited extent. Although 15 or more years have already passed since the launching of this drug and in the meanwhile various compounds have been clinically developed, a new therapeutic agent for ALS still remains to be developed. Thus, there is a strong unmet medical need for the development of therapeutic agents effective against ALS. An object of the present invention is to provide a therapeutic agent for ALS, for which no effective drug exists, the therapeutic agent being capable of suppressing the pathological progression of ALS and effectively treating this disease.

### Solution to Problem

The present inventors have conducted diligent studies on therapeutic agents useful against ALS for which no fully (or sufficiently) effective drug exists, particularly drugs capable of more effectively treating ALS compared with the only one existing drug riluzole. As a result, the present inventors have completed the present invention by finding that GHS-R agonists exhibit a more effective motor neuron protective effect than that of the existing drug. Specifically, the present invention includes the following aspects:
(1) A therapeutic agent for use in treating amyotrophic lateral sclerosis comprising a growth hormone secretagogue receptor agonist or a pharmaceutically acceptable salt thereof as an active ingredient, wherein the growth hormone secretagogue receptor agonist is ghrelin, GHRP-6, or anamorelin, for administration to an individual having amyotrophic lateral sclerosis with non-serious dysphagia.
(2) The therapeutic agent for use according to (1), wherein the individual is also unresponsive or insufficiently responsive to an existing therapeutic agent for amyotrophic lateral sclerosis.
(3) The therapeutic agent for use according to (1) or (2), wherein the therapeutic agent is used in combination with an existing therapeutic agent for amyotrophic lateral sclerosis.
(4) The therapeutic agent for use according to (2) or (3), wherein the existing therapeutic agent for amyotrophic lateral sclerosis is riluzole.
(5) The therapeutic agent for use according to any one of (1) to (4), wherein the therapeutic agent is administered by a subcutaneous injection.
(6) The therapeutic agent for use according to any one of (1) to (5), wherein the ghrelin is a peptide compound comprising the amino acid sequence of SEQ ID NO: 1 in which the 3rd amino acid residue from the amino terminus is a modified amino acid residue with a fatty acid introduced in the side chain of the amino acid residue, or a peptide compound comprising an amino acid sequence of SEQ ID NO: 1 with the deletion, substitution and/or addition of one or several amino acid residues at position 5 to 28 from the amino terminus of SEQ ID NO: 1 in which the 3rd amino acid residue from the amino terminus is a modified amino acid residue with a fatty acid introduced in the side chain of the amino acid residue, and having the activity of elevating an intracellular calcium ion concentration through binding to a growth hormone secretagogue receptor.
(7) The therapeutic agent for use according to (6), wherein the ghrelin is a peptide compound comprising the amino acid sequence of SEQ ID NO: 1 in which the 3rd amino acid residue from the amino terminus is a modified amino acid residue with a fatty acid introduced at a hydroxy group in the side chain of the amino acid residue.
(8) The therapeutic agent for use according to (7), wherein the ghrelin is a peptide compound comprising the amino acid sequence of SEQ ID NO: 1 in which the hydroxy group in the side chain of the 3rd amino acid residue from the amino terminus is acylated with a n-octanoyl group.
(9) A growth hormone secretagogue receptor agonist or a pharmaceutically acceptable salt thereof for use in treating amyotrophic lateral sclerosis by administration to an individual having amyotrophic lateral sclerosis with non-serious dysphagia, wherein the growth hormone secretagogue receptor agonist is ghrelin, GHRP-6, or anamorelin.
(10) The growth hormone secretagogue receptor agonist or the pharmaceutically acceptable salt thereof for use according to (9), wherein the individual is also unresponsive or insufficiently responsive to an existing therapeutic agent for amyotrophic lateral sclerosis.
(11) The growth hormone secretagogue receptor agonist or the pharmaceutically acceptable salt thereof for use according to (9) or (10), wherein in the treatment, the growth hormone secretagogue receptor agonist or the pharmaceutically acceptable salt thereof is used in combination with an existing therapeutic agent for amyotrophic lateral sclerosis.
(12) The growth hormone secretagogue receptor agonist or the pharmaceutically acceptable salt thereof according to (10) or (11), wherein the existing therapeutic agent for amyotrophic lateral sclerosis is riluzole.
(13) The growth hormone secretagogue receptor agonist or the pharmaceutically acceptable salt thereof according to any one of (9) to (12), wherein the administration of the growth hormone secretagogue receptor agonist or the pharmaceutically acceptable salt thereof to the individual is administration in the form of a subcutaneous injection.
(14) The growth hormone secretagogue receptor agonist or the pharmaceutically acceptable salt thereof according to any one of (9) to (13), wherein the ghrelin is a peptide compound comprising the amino acid sequence of SEQ ID NO: 1 in which the 3rd amino acid residue from the amino terminus is a modified amino acid residue with a fatty acid introduced in the side chain of the amino acid residue, or a peptide compound comprising an amino acid sequence of SEQ ID NO: 1 with the deletion, substitution and/or addition of one or several amino acid residues at position 5 to 28 from the amino terminus of SEQ ID NO: 1 in which the 3rd amino acid residue from the amino terminus is a modified amino acid residue with a fatty acid introduced in the side chain of the amino acid residue, and having the activity of elevating an intracellular calcium ion concentration through binding to a growth hormone secretagogue receptor.
(15) The growth hormone secretagogue receptor agonist or the pharmaceutically acceptable salt thereof according to (14), wherein the ghrelin is a peptide compound comprising the amino acid sequence of SEQ ID NO: 1 in which the 3rd amino acid residue from the amino terminus is a modified amino acid residue with a fatty acid introduced at a hydroxy group in the side chain of the amino acid residue.
(16) The growth hormone secretagogue receptor agonist or the pharmaceutically acceptable salt thereof according to (15), wherein the ghrelin is a peptide compound comprising the amino acid sequence of SEQ ID NO: 1 in which the hydroxy group in the side chain of the 3rd amino acid residue from the amino terminus is acylated with a n-octanoyl group.

### Advantageous Effects of Invention

The GHS-R agonist of the present invention, which is ghrelin, GHRP-6, or anamorelin, has the effect of prolonging survival periods by suppressing the pathological progression of ALS, and as such, is very useful in the treatment of ALS for which no effective drug exists. Moreover, the GHS-R agonist of the present invention is further useful because it has the effect of suppressing muscle weakness by inhibiting the decrease in the number of motor neurons characteristic of the pathological onset and progression of ALS and thereby remarkably suppressing the death of the cells. The GHS-R agonist of the present invention is superior in these effects to riluzole, an existing therapeutic agent for ALS, and meets the longtime unmet medical need, because the GHS-R agonist can prolong survival periods even if administered from the time when riluzole is no longer effective. Furthermore, the GHS-R agonist of the present invention has a motor neuron protective effect and suppresses muscle weakness, leading not only to prolonged survival periods but to improved quality of life during the survival periods. In addition, because of this motor neuron protective effect, the GHS-R agonist is useful against motor neuron diseases or disorders other than ALS, for example, spinal muscular atrophy.

### Description of Embodiments

A peptide or non-peptide compound known in the art can be used as the GHS-R agonist of the present invention. Examples of the peptide compound disclosed herein include the endogenous ligand ghrelin as well as pralmorelin (GHRP-2), GHRP-6, hexarelin, and ipamorelin. Examples of the non-peptide compound disclosed herein include ibutamoren mesilate (MK-0677), ulimorelin (TZP-101), anamorelin (RC-1291), macimorelin (AEZS-130), capromorelin (CP-424391), and SM-130686. Among these peptide compounds, ghrelin is particularly desirable. Among these non-peptide compounds, anamorelin is particularly desirable.

Examples of the ghrelin can include human-derived ghrelin and ghrelin derived from any other animal such as rats, mice, pigs, and cattle, and their derivatives (see e.g., International Publication No. WO01/07475). When the recipient individual is a human, human-derived ghrelin is desirably used. The human-derived ghrelin is a peptide compound composed of 28 amino acids (SEQ ID NO: 1) in which a hydroxy group in the side chain of the 3rd amino acid (serine) residue counted from the amino terminus is acylated with a fatty acid (n-octanoyl group).

The endogenous ligand ghrelin is a hormone existing *in vivo* and can serve as a particularly highly safe therapeutic agent for ALS because its safety in administration to humans has already been confirmed.

A peptide having an amino acid sequence derived from the amino acid sequence represented by SEQ ID NO: 1 by the deletion, substitution, and/or addition of one or several amino acids selected from the 5th to 28th amino acid residues counted from the amino terminus in which the 3rd amino acid (serine) residue counted from the amino terminus is a modified amino acid residue with a fatty acid introduced in the side chain (hydroxy group) of the amino acid residue, and having the activity of elevating an intracellular calcium ion concentration through binding to GHS-R can be used as a ghrelin derivative (see International Publication No. WO01/07475).

In this context, the term "several" used herein means 1 to 8, 1 to 7, 1 to 6, 1 to 5, 1 to 4, 1 to 3, or 1 or 2.

Desirably, the amino acid sequence of the ghrelin derivative has 70%, preferably 80%, more preferably 90%, particularly preferably 95%, most preferably 97% homology to the naturally occurring amino acid sequence. This holds true for a splicing variant (SEQ ID NO: 2) of human-derived ghrelin composed of 27 amino acids.

As for the activity of the ghrelin derivative, agonistic activity against GHS-R or biological activity described in the above publication can be used as an index. On the basis of the index, the ghrelin derivative of interest can be selected. For example, the agonistic activity against GHS-R can be examined with an intracellular calcium ion concentration as an index. This index can be measured by use of a method known in the art, and, for example, FLIPR (Fluorometric Imaging Plate Reader, Molecular Devices, LLC) based on change in the fluorescence intensity of Fluo-4 AM (Molecular Probes) caused by change in calcium ion concentration can be used. Also, the *in vivo* orexigenic activity of the peptide or non-peptide compound having calcium concentration-elevating activity can be confirmed by use of a method known in the art. For example, in order to confirm its orexigenic effect on healthy mice, the peptide or non-peptide compound having calcium concentration-elevating activity is administered subcutaneously or intraperitoneally to the animals, and their food intakes 1 hour after the administration can be compared with a vehicle-administered group.

D-Alanyl-D-(2-naphthyl)alanyl-L-alanyl-L-tryptophyl-D-phenylalanyl-L-lysinamide (U.S. Patent No. 5,663,146) can be used as pralmorelin (GHRP-2).

L-Histidyl-D-tryptophyl-L-alanyl-L-tryptophyl-D-phenylalanyl-L-lysinamide (Endocrinology (1984), vol. 114 (No. 5), p. 1537-1545) can be used as GHRP-6.

L-Histidyl-2-methyl-D-tryptophyl-L-alanyl-L-tryptophyl-D-phenylalanyl-L-lysinamide (U.S. Patent No. 5,646,301) can be used as hexarelin.

α-Methylalanyl-L-histidyl-D-β-(2-naphthyl)-L-alanyl-D-phenylalanyl-L-lysinamide (Journal of Medicinal Chemistry (1998), vol. 41, p. 3699-704) can be used as ipamorelin.

2-Amino-2-methyl-N-[(1R)-2-(1-methanesulfonylspiro[indoline-3,4'-piperidin]-1'-yl)-2-oxo-1-(phenylmethoxymethyl)ethyl]propanamide mesilate (U.S. Patent No. 5,536,716) can be used as ibutamoren mesilate (MK-0677).

5(S)-Cyclopropyl-11(R)-(4-fluorobenzyl)-2(R),7,8(R)-trimethyl-2,3,4,5,6,7,8,9,10,11,13,14,15,16-tetradecahydro-1,4,7,10,13-benzoxatetraazacyclooctadecyne-6,9,12-trione (U.S. Patent No. 7,476,653) can be used as ulimorelin (TZP-101).

2-Amino-N-{(1R)-2-[3-benzyl-3-(N,N',N'-trimethylhydrazinocarbonyl)piperidin-1-yl]-1-((1H-indol-3-yl)-2-oxoethyl)-2-methylpropionamide} (U.S. Patent No. 6,576,648) can be used as anamorelin.

2-Methylalanyl-N-[1(R)-formamido-2-(1H-indol-3-yl)ethyl]-D-tryptophanamide (U.S. Patent No. 6,861,409) can be used as macimorelin (AEZS-130).

2-Amino-N-[2-[3a(R)-benzyl-2-methyl-3-oxo-3,3a,4,5,6,7-hexahydro-2H-pyrazolo[4,3-c]pyridin-5-yl]-1(R)-(benzyloxymethyl)-2-oxoethyl]isobutyramide (EP Patent No. 0869968) can be used as capromorelin (CP-424391).

(+)-3(S)-(2-Chlorophenyl)-1-[2-(diethylamino)ethyl]-3-hydroxy-2-oxo-4-(trifluoromethyl)-2,3-dehydro-1H-indole-6-carboxyamide hydrochloride (U.S. Patent No. 6,576,656) can be used as SM-130686.

The salt of the GHR-S agonist that can be used in the present invention is preferably a pharmaceutically acceptable salt. Examples thereof include salts with inorganic bases, salts with organic bases, salts with inorganic acids, salts with organic acids, and salts with basic or acidic amino acids.

Preferred examples of the salts with inorganic bases include: alkali metal salts such as sodium salt and potassium salt; alkaline earth metal salts such as calcium salt and magnesium salt; and aluminum salt and ammonium salt.

Preferred examples of the salts with organic bases include trimethylamine, triethylamine, pyridine, picoline, ethanolamine, diethanolamine, triethanolamine, dicyclohexylamine, and N,N'-dibenzylethylenediamine.

Preferred examples of the salts with inorganic acids include salts with hydrochloric acid, hydrobromic acid, nitric acid, sulfuric acid, and phosphoric acid.

Preferred examples of the salts with organic acids include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid.

Preferred examples of the salts with basic amino acids include salts with arginine, lysine, and ornithine. Preferred examples of the salts with acidic amino acids include salts with aspartic acid and glutamic acid.

Among these salts, sodium salt or potassium salt is most preferable.

The GHS-R agonist for use according to the present invention can be obtained by a routine method. For example, the GHS-R agonist can be isolated from a natural raw material or can be produced by a recombinant DNA technique and/or a chemical synthesis technique.

In the case of producing the peptide compound, for use according to the present invention, using a recombinant DNA technique, examples of vectors for incorporation of a gene of interest encoding the peptide compound according to the present invention include *E. coli* vectors (pBR322, pUC18, pUC19, etc.), *Bacillus subtilis* vectors (pUB110, pTP5, pC194, etc.), yeast vectors (YEp, YRp, and YIp types), and animal cell vectors (retrovirus, vaccinia virus, etc.). Any other vector can be used as long as the vector can allow the host cells to carry the gene of interest stably. The vectors are transferred to appropriate host cells. A method described in, for example, Molecular Cloning: A Laboratory Manual (Cold Spring Harbor Laboratory Press, 1989) can be used as a method for incorporating the gene of interest into plasmids or a method for transferring the plasmids to host cells.

In the plasmids, a promoter is functionally connected upstream of the gene in order to express the peptide gene of interest.

The promoter used may be any promoter suitable for host cells for use in the expression of the gene of interest. When the host cells to be transformed are, for example, bacterial cells of the genus *Escherichia*, a lac promoter, trp promoter, lpp promoter, λPL promoter, recA promoter, or the like can be used. For host cells of the genus *Bacillus*, an SPO1 promoter, SPO2 promoter, or the like can be used. For yeast cells, a GAP promoter, PHO5 promoter, ADH promoter, or the like can be used. For animal cells, an SV40-derived promoter, retrovirus-derived promoter, or the like can be used.

The thus-obtained vectors containing the gene of interest are used in the transformation of host cells. Bacterial cells (e.g., the genera *Escherichia* and *Bacillus*), yeast cells (e.g., the genera *Saccharomyces*, *Pichia*, and *Candida*), animal cells (e.g., CHO cells and COS cells), or the like can be used as host cells. A liquid medium is appropriate as a culture medium. Particularly preferably, the medium contains a carbon source, a nitrogen source, and the like necessary for the growth of transformed cells to be cultured. If desired, the medium can be further supplemented with vitamins, a growth stimulant, serum, and the like.

After culturing, the peptide compound for use according to the present invention is separated and purified from the cultures by a routine method. For example, in order to extract the substance of interest from the cultured microbial cells or animal cells, these cells thus cultured are collected, then suspended in a buffer solution containing a protein denaturant (guanidine hydrochloride, etc.), and disrupted ultrasonically, for example, followed by centrifugation. Next, the substance of interest can be purified from the supernatant by an appropriate combination of separation and purification methods such as gel filtration, ultrafiltration, dialysis, SDS-PAGE, and various chromatography techniques in consideration of the molecular weight, solubility, charge (isoelectric point), affinity, etc. of the substance of interest.

In the case of producing the peptide compound for use according to the present invention using a chemical synthesis technique, for example, amino acids with protective groups are condensed by a liquid-phase method and/or a solid-phase method to extend a peptide chain. All of the protective groups are removed with an acid. The obtained crude product is purified by a purification method known in the art to obtain the peptide compound for use according to the present invention.

The ghrelin and its derivative for use according to the disclosure herein can also be obtained by routine methods. For example, the ghrelin and its derivative for use according to the disclosure herein can be isolated or purified from a natural raw material or can be produced by a recombinant DNA technique and/or a chemical synthesis technique.

As the ghrelin contains an amino acid residue modified (acylated) at its side chain, the amino acid residue can be modified (acylated) through a modification reaction according to an approach known in the art. For example, in a production method using a recombinant DNA technique, host cells transformed with expression vectors containing DNA encoding ghrelin or its derivative are cultured, and the peptide of interest can be collected from the cultures to obtain the ghrelin or its derivative for use according to the disclosure herein. The host cells can be screened to obtain the modified (acylated) compound of the peptide of interest within the cells. For example, cells having processing protease activity capable of cleaving a precursor polypeptide of the peptide at a suitable position and having the activity of acylating the serine residue in the peptide are desirably used for directly producing the fatty acid-modified (acylated) peptide compound. Such host cells having the processing protease activity and the serine-acylating activity can be selected by: transforming the host cells with expression vectors containing cDNA encoding the precursor polypeptide; and confirming that the transformed cells produce the fatty acid-modified peptide compound having calcium concentration-elevating activity or growth hormone secretagogue activity.

In a production method using a chemical synthesis technique, for example, amino acids with protective groups are condensed by a liquid-phase method and/or a solid-phase method to extend a peptide chain. All of the protective groups are removed with an acid. The obtained crude product can be purified by the above purification method to obtain the ghrelin or its derivative for use according to the disclosure herein. The side chain of the amino acid located at the position of interest can be selectively acylated with an acylating enzyme or acyltransferase.

Alternatively, a recombinant DNA technique and a chemical synthesis technique may be used in combination in a production method. Such a production method can involve producing a fragment containing the modified amino acid residue by chemical synthesis, while producing other fragments free from the modified amino acid residue by a recombinant DNA technique, and then fusing these fragments (see International Publication Nos. WO01/07475 and WO03/084983).

The other compounds that can be used in the present disclosure, i.e., pralmorelin (GHRP-2), GHRP-6, hexarelin, ipamorelin, ibutamoren mesilate (MK-0677), ulimorelin, anamorelin, macimorelin, capromorelin, and SM-130686 can also be produced by methods known in the art, including the above methods.

ALS animal models (SOD1^{G93A} mice) were raised under restricted feeding, and GHS-R agonist was administered to the animals such that the GHS-R agonist exhibited no orexigenic effect. In this case, neither a muscle weakness suppressive effect nor a motor neuron death suppressive effect was observed, though a decrease in skeletal muscle mass was inhibited. Thus, the GHS-R agonist exhibits a muscle weakness suppressive effect and/or a survival period-prolonging effect in individuals in which the GHS-R agonist exerts its orexigenic effect. Accordingly, the recipient of the present invention is, among ALS-affected individuals, an individual whose food intake can be increased by the administration of the GHS-R agonist, in short, an "individual with non-serious dysphagia" who is capable of orally ingesting as much food as desired by the individual. Since the GHS-R agonist can exert its therapeutic effect by administration even from the time when riluzole is no longer effective, the individual may also be "unresponsive or insufficiently responsive to riluzole".

### (1) Individual with non-serious dysphagia

The term "individual with non-serious dysphagia" refers to an individual capable of orally ingesting food at a desired intake. This individual can eat as much as the individual wants. In other words, the individual may receive the contents of a diet rendered easy-to-eat (processed food in a paste form, etc.), use an eating tool for rendering food easy-to-eat, and receive care to help the individual eat (e.g., assistance in use of an eating tool) as long as the swallowing function of the individual is maintained.

The "non-serious dysphagia" can be determined according to the Revised ALS Functional Rating Scale (hereinafter, referred to as ALSFRS-R; Journal of the Neurological Sciences (1999), vol. 169 (No. 1-2), p. 13-21), a clinical evaluation index established worldwide. The non-serious dysphagia corresponds to score 2 (dietary consistency changes) or higher, preferably score 3 (Early eating problems - occasional choking) or higher, more preferably score 4 (normal eating habits) under the measure of swallowing in the functional rating scale.

In the case of using ghrelin as the GHS-R agonist, ghrelin exhibits a muscle weakness suppressive effect and/or a survival period-prolonging effect, as described in detail in the Examples, even under conditions where SOD1^{G93A} mice are assisted in eating by scattering feed on the floor at the age of 18 weeks or later when the mice have difficulty in taking feed from a bait box. Specifically, ghrelin can suppress the muscle weakness of the ALS animal models and prolong their survival periods, when administered under conditions where its orexigenic effect can be achieved.

### (2) Individual unresponsive or insufficiently responsive to existing therapeutic agent for amyotrophic lateral sclerosis

The term "unresponsive" in relation to an existing therapeutic agent for amyotrophic lateral sclerosis (therapeutic agent for ALS) refers to a state where the therapeutic effect of the existing therapeutic agent for ALS previously or currently used is not seen or a state where the effect is not maintained, i.e., a state where the pathological progression is not suppressed by the administration of the existing therapeutic agent for ALS. The unresponsiveness to treatment with the existing therapeutic agent for ALS is evaluated by the examination of one or more clinical evaluation indexes in ALSFRS-R mentioned above. Thus, the unresponsiveness to the existing therapeutic agent for ALS can be determined by a clinician skilled in the treatment of ALS. For example, a clinician evaluates the status of an individual according to ALSFRS-R at a medical examination every 3 months. When the rate of change (decreased score / period) in the rating scale for a predetermined period (e.g., 3 months) after the start of treatment with the existing therapeutic agent for ALS is equivalent to or greater than that in the rating scale for a predetermined period (e.g., 6 months) before the start of the treatment with the existing therapeutic agent for ALS, the existing therapeutic agent for ALS is not effective for the individual. Thus, the individual is confirmed to be unresponsive to the existing therapeutic agent for ALS.

The individual unresponsive to treatment with the existing therapeutic agent for ALS also includes an individual on which the existing therapeutic agent has previously produced its therapeutic effect in response to the treatment, but no longer produces a similar effect by the treatment at the moment.

The term "insufficiently responsive" to the existing therapeutic agent for ALS refers to a state where the pathological progression is not sufficiently suppressed by treatment with the existing therapeutic agent for ALS due to the inadequate therapeutic effect of the existing therapeutic agent for ALS. The insufficient responsiveness to treatment with the existing therapeutic agent for ALS is evaluated by the examination of one or more clinical evaluation indexes in ALSFRS-R as mentioned above. Thus, the insufficient responsiveness to the existing therapeutic agent for ALS can be determined by a clinician skilled in the treatment of ALS. For example, a clinician evaluates the status of an individual according to ALSFRS-R at a medical examination every 3 months. When the rate of change (decreased score / period) in the rating scale for a predetermined period (e.g., 3 months) after the start of treatment with the existing therapeutic agent for ALS exhibits less than 30% alleviation compared with that in the rating scale for a predetermined period (e.g., 6 months) before the start of the treatment with the existing therapeutic agent for ALS, the existing therapeutic agent for ALS is insufficiently effective for the individual. Thus, the individual is confirmed to be "insufficiently responsive" to the existing therapeutic agent for ALS.

The term "treatment" used herein refers to the control, reduction, or prevention of the pathological progression of ALS. The treatment includes the control, reduction, or prevention of the pathological progression of progressive degeneration of motor neurons, denervation of muscle fibers, muscle wasting, muscle weakness, contracture, or paralysis, and the prolonging of survival periods.

The "therapeutic effect" described herein can be determined by a method known in the art. The therapeutic effect can be determined by the evaluation of, for example, a functional status based on ALSFRS-R, respiratory functions based on Forced Vital Capacity (FVC), or muscle strength based on the Medical Research Council (MRC) Scale.

Any other method may be used as long as the degree of ALS symptoms can be determined by the method. The therapeutic effect can be determined on the basis of, for example, grasping power, back muscle strength, the ability or inability to ambulate independently, the presence or absence of tubal feeding, the time period to tubal feeding (the starting date of the time period can be arbitrarily set and may be, for example, the day on which the administration of the therapeutic agent for ALS of the present invention or the existing therapeutic agent for ALS is started or the day when ALS symptoms such as muscle weakness are observed for the first time), the presence or absence of tracheotomy, the presence or absence of placement of a respirator, the time period to intubation for the placement of a respirator or tracheotomy (the starting date of the time period can be arbitrarily set and may be, for example, the day on which the administration of the therapeutic agent for ALS of the present invention or the existing therapeutic agent for ALS is started or the day when ALS symptoms such as muscle weakness are observed for the first time), or a survival period (the starting date of the time period can be arbitrarily set and may be, for example, the day on which the administration of the therapeutic agent for ALS of the present invention or the existing therapeutic agent for ALS is started or the day when ALS symptoms such as muscle weakness are observed for the first time).

The "therapeutic effect" of the drug may be determined using any of these methods after the completion of the dosing period of the therapeutic agent for ALS of the present invention or the existing therapeutic agent for ALS or during the dosing period thereof.

The therapeutic agent for ALS comprising the GHS-R agonist according to the present invention as an active ingredient may be administered alone to a recipient individual or may be administered in combination with an additional drug to a recipient individual.

The term "(administered) in combination" or "used in combination" used herein refers to the administration of two or more types of drugs to one individual. These drugs may be administered simultaneously or almost simultaneously (e.g., within 1 hour) or may be administered in a staggered manner at an interval of several hours. For example, a first drug is administered every day, immediately followed by the administration of a second drug. Typically, the first and second drugs are administered at times suitable for these drugs to exert their effects. In the case of using, for example, the therapeutic agent for ALS comprising the GHS-R agonist ghrelin as an active ingredient in combination with an existing therapeutic agent for ALS, the existing therapeutic agent for ALS is administered before meals every morning and every evening, immediately followed by the administration (e.g., subcutaneous administration) of the therapeutic agent for ALS comprising ghrelin as an active ingredient, or vice versa. In this way, these drugs can be used in combination.

Since ghrelin exhibits a muscle weakness suppressive effect and/or a survival period-prolonging effect under conditions where the existing therapeutic agent for ALS is ineffective, this drug is effective for an ALS-affected individual unresponsive or insufficiently responsive to the existing therapeutic agent for ALS and is also expected to have efficacy on an ALS-affected individual insufficiently responsive to the existing therapeutic agent for ALS when used in combination with the existing therapeutic agent for ALS. Examples of the existing therapeutic agent for ALS can include riluzole.

The GHS-R agonist or the pharmacologically acceptable salt thereof that can be used in the present invention can be administered orally or parenterally in the form of a solid preparation (tablets, capsules, granules, fine granules, powders, etc.) or a liquid preparation (syrups, injections, etc.) supplemented with pharmaceutically acceptable carriers.

Various organic or inorganic carrier substances routinely used as pharmaceutical materials are used as the pharmaceutically acceptable carriers. The solid preparation is supplemented with an excipient, a lubricant, a binder, a disintegrant, and the like. The liquid preparation is supplemented with a solvent, a solubilizer, a suspending agent, a tonicity agent, a pH adjuster, a buffering agent, a soothing agent, and the like. If necessary, pharmaceutical additives such as an antiseptic, an antioxidant, a colorant, and a sweetener may be further used in these preparations.

Preferred examples of the excipient include lactose, saccharose, D-mannitol, starch, crystalline cellulose, and light anhydrous silicic acid. Preferred examples of the lubricant include magnesium stearate, calcium stearate, talc, and colloidal silica.

Preferred examples of the binder include crystalline cellulose, saccharose, D-mannitol, dextrin, hydroxypropylcellulose, hydroxypropylmethylcellulose, and polyvinylpyrrolidone.

Preferred examples of the disintegrant include starch, carboxymethylcellulose, calcium carboxymethylcellulose, sodium croscarmellose, and sodium carboxymethyl starch.

Preferred examples of the solvent include injectable water, alcohols, propylene glycol, Macrogol, sesame oil, and corn oil.

Preferred examples of the solubilizer include polyethylene glycol, propylene glycol, D-mannitol, benzyl benzoate, ethanol, trisaminomethane, cholesterol, triethanolamine, sodium carbonate, and sodium citrate.

Preferred examples of the suspending agent include: surfactants such as stearyl triethanolamine, sodium lauryl sulfate, lauryl aminopropionic acid, lecithin, benzalkonium chloride, benzethonium chloride, and glycerin monostearate; and hydrophilic polymers such as polyvinyl alcohol, polyvinylpyrrolidone, sodium carboxymethylcellulose, methylcellulose, hydroxymethylcellulose, hydroxyethylcellulose, and hydroxypropylcellulose.

Preferred examples of the tonicity agent include sodium chloride, glycerin, and D-mannitol.

Preferred examples of the buffering agent include phosphate, acetate, carbonate, and citrate buffer solutions.

Preferred examples of the soothing agent include benzyl alcohol.

Preferred examples of the antiseptic include p-hydroxybenzoate esters, chlorobutanol, benzyl alcohol, phenethyl alcohol, dehydroacetic acid, and sorbic acid.

Preferred examples of the antioxidant include a sulfite and ascorbic acid.

Examples of dosage forms suitable for parenteral administration can include injections, drops, suppositories, percutaneous absorption formulations, transmucosal absorption formulations, and inhalants for intravenous administration, intracutaneous administration, subcutaneous administration, or intramuscular administration. Examples of dosage forms suitable for oral administration can include capsules, tablets, and syrups. When the active ingredient in the therapeutic agent of the present invention is a peptide compound, its dosage form is preferably a dosage form suitable for parenteral administration, for example, an injection, drops, or an inhalant. Various such dosage forms are known to those skilled in the art. Those skilled in the art can appropriately select a dosage form suitable for the desired administration route and can produce a preparation in the form of a pharmaceutical composition using, if necessary, one or two or more pharmaceutical additives that may be used in the art. For example, the therapeutic agent in the form of an injection or drops can be prepared by: dissolving the active ingredient GHS-R agonist together with one or two or more pharmaceutical additives such as a tonicity agent, a pH adjuster, a soothing agent, and an antiseptic in injectable distilled water; and sterilizing the solution. Alternatively, the therapeutic agent in the form of an injection or drops may be provided as a freeze-dried therapeutic agent. Such a preparation can be dissolved by the addition of injectable distilled water or saline before use and used as an injection or drops.

Since human ghrelin exhibits a muscle weakness suppressive effect and/or a survival period-prolonging effect in ALS animal models by subcutaneous administration, the ghrelin or its derivative, or a pharmacologically acceptable salt of the ghrelin or the derivative, or the peptide or non-peptide GHS-R agonist can be administered in the form of an injection such as a subcutaneous injection.

When the active ingredient is a peptide compound, this compound may be orally administered in the form of a preparation unsusceptible to digestion in the gastrointestinal tract, for example, in the form of a microcapsule comprising the active ingredient peptide enclosed in a liposome. Another possible administration method involves absorption through a mucosal membrane other than the gastrointestinal mucosa, such as rectal mucosa, nasal mucosa, or hypoglossal mucosa. In this case, the compound can be administered in the form of a suppository, a nasal spray, an inhalant, or a sublingual tablet to the individual. Alternatively, a preparation improved in terms of the retention of the peptide in blood by the adoption of, for example, a controlled-release preparation or a sustained release preparation containing a polysaccharide such as dextran or a biodegradable polymer typified by polyamine or PEG as a carrier can also be used in the present invention.

If a non-peptide compound is used as the active ingredient in oral administration, the compound can be tabletted, charged into hard capsules in the form of powders or pellets, or prepared in the form of a troche, together with solid carriers such as an excipient, a lubricant, a binder, and a disintegrant. The amount of the solid carriers may vary widely and is usually approximately 25 mg to approximately 1 g. In the case of using liquid carriers, a preparation composed of the active ingredient and the liquid carriers can be administered in the form of a syrup, an emulsion, a soft capsule, or an aqueous or nonaqueous liquid suspension or solution.

The dose of the GHS-R agonist that can be used as the active ingredient in the therapeutic agent for ALS according to the present invention can be appropriately selected according to the age, body weight, degree of symptoms, and administration route of the individual (patient). The upper limit of the daily dose thereof to a human adult as the ALS-affected individual is generally, for example, approximately 100 mg/kg or smaller, preferably approximately 10 mg/kg or smaller, more preferably 1 mg/kg or smaller, in terms of the upper limit of the weight of the substance. The lower limit of the daily dose thereof is, for example, approximately 0.1 µg/kg or larger, preferably 1 µg/kg or larger, more preferably 10 µg/kg or larger. Since the substance that can be used as the active ingredient in the pharmaceutical composition according to the present invention suppresses the pathological progression of ALS under conditions where its orexigenic effect is achieved, its administration period terminates when the ALS-affected individual manifests prominent dysphagia which is no longer non-serious, and requires an involuntary oral ingestion approach such as tubal feeding. The substance can be administered repeatedly or continuously approximately once or twice a day for several months to several years up to this stage. For the repeated administration of the substance, the substance is desirably administered before meals every morning and/or every evening.

### Examples

Hereinafter, the present invention will be described specifically with reference to Examples. However, these Examples are given merely for the purpose of illustrating the present invention and are not intended to limit the present invention.

In the Examples below, the ALS animal models used were SOD1^{G93A} mice. First, B6SJL-Tg(SOD1-G93A)1Gur/J mice (SOD1^{G93A} mice) and wild-type mice of the same line thereas were purchased from The Jackson Laboratory (ME, USA) and mated with each other to obtain offspring. The obtained SOD1^{G93A} mice were further bred to wild-type littermates (WT mice). The newly born SOD1^{G93A} mice were used in experiments. In some Examples, the WT mice were also used.

The SOD1^{G93A} mice and the WT mice were freely given tap water and rodent standard feed pellet (CRF-1, 13.6% of the total calorie is derived from fat, 3570 kcal/kg, Oriental Yeast Co., Ltd.) while being raised. The SOD1^{G93A} mice were assisted in eating by scattering feed on the floor at the age of 18 weeks or later when the mice had difficulty in taking feed from a bait box due to a decline in lower limb functions.

### <Example 1> Comparison of body weight and forelimb muscle strength between 10-week-old SOD1^{G93A} mice and WT mice

The SOD1^{G93A} mice have selective death of motor neurons at the stage of maturation and manifest skeletal muscle wasting or loss of muscle strength, eventually leading to death, as in human ALS. Thus, the body weights and forelimb muscle strengths of 10-week-old SOD1^{G93A} mice were first compared with those of WT mice to confirm the onset of ALS at this age.

### 1. Materials and Methods

In this Example, 10-week-old WT and SOD1^{G93A} mice were used, and their body weights and forelimb muscle strengths were measured. The forelimb muscle strengths were measured using a rat/mouse simple sthenometer; 200 g scale (O'HARA & CO., LTD.).

### 2. Results

The body weight and forelimb muscle strength of each group are shown in Table 1.

The SOD1^{G93A} mice exhibited values as low as an average body weight of approximately 1 g smaller and as significantly low as a forelimb muscle strength of approximately 0.1 N lower on average than those of the WT mice. This demonstrated that the SOD1^{G93A} mice had already lost their muscle strength and developed ALS at the age of 10 weeks.

**[Table 1]**

| | WT mouse | SOD1^{G93A} mouse |
|---|---|---|
| Body weight (g) | 25.2 ± 0.7 (18) | 24.1 ± 0.7 (10) |
| Forelimb muscle strength (N) | 1.04 ± 0.04 (18) | 0.91 ± 0.04 (10)* |

| | | |
|---|---|---|
| The numerical values were indicated by mean ± SE (the number of cases) *: P < 0.05. vs. WT mice (Student's t test) | | |

### <Example 2> Effect of riluzole on SOD1^{G93A} mice

### 1. Materials and Methods

Riluzole, an existing therapeutic agent for ALS, has been reported to exhibit a life-prolonging effect when administered to SOD1^{G93A} mice from ages of 4 weeks or 7 weeks and in either case before the onset of ALS (Amyotrophic Lateral Sclerosis (2009), vol. 10, p. 85-94; and Annals of Neurology (1996), vol. 39, p. 147-157). In Example 1, the SOD1^{G93A} mice were confirmed to manifest muscle weakness as a symptom of ALS at the age of 10 weeks.

In this Example, the 10-week-old SOD1^{G93A} mice were divided into 2 groups: a vehicle (saline solution) group and a riluzole group. A saline solution or riluzole (Sigma-Aldrich Corp, 16 mg/kg) was intraperitoneally administered thereto once a day until death, and the survival period of each individual was analyzed. The dose of riluzole was set according to the previous report (Amyotrophic Lateral Sclerosis (2009), vol. 10, p. 85-94).

### 2. Results

The average survival period of each group is shown in Table 2.

The vehicle group and the riluzole group had equivalent average survival periods. Thus, riluzole was not confirmed to have a life-prolonging effect under the condition of administration from the age of 10 weeks.

**[Table 2]**

| | Vehicle group | Riluzole group |
|---|---|---|
| Survival period (day) | 137 ± 3 (7) | 134 ±3 (6) |

| | | |
|---|---|---|
| The numerical values were indicated by mean ± SE (the number of cases) | | |

### <Example 3> Effect of human-derived ghrelin (hereinafter, referred to as human ghrelin) on SOD1^{G93A} mice - (1): Effects on skeletal muscle mass, muscle strength, and survival period by continuous subcutaneous administration

In Example 2, riluzole was confirmed to exhibit no survival period-prolonging effect when administered to SOD1^{G93A} mice from the age of 10 weeks. The effect of human ghrelin (SEQ ID NO: 1) was examined under such conditions where the SOD1^{G93A} mice already manifested muscle weakness as a symptom of ALS and riluzole was not effective for prolonging their survival periods.

### 1. Materials and Methods

In the experiment, 10-week-old SOD1^{G93A} mice were used, which were divided into 2 groups: a vehicle group and a human ghrelin group. Human ghrelin (50 µg/day, approximately 2 mg/kg/day) was dissolved in a vehicle (saline solution) to prepare a dosing solution. An osmotic pump (ALZET(R) MINI-OSMOTIC PUMP MODEL 1004, DURECT Corporation) filled with the dosing solution or a saline solution was subcutaneously implanted into the back of each mouse for continuous subcutaneous administration. The administration was started from the age of 10 weeks and continued to the live individuals with the osmotic pump replaced with a fresh one every 4 weeks.

Their body weight and feed weight was measured before the start of administration and after 8-weeks of administration to calculate the amount of change in body weight and the food intake. After 8-weeks of administration, the lower-body skeletal muscle masses of the mice were measured using X-ray CT (Latheta LCT-200, Hitachi Aloka Medical, Ltd.). The plasma total cholesterol concentrations were measured with Cholesterol E - Test Wako (Wako Pure Chemical Industries, Ltd.) using plasma separated from blood collected from the tail vein after 8-weeks of administration. The forelimb muscle strengths were measured using a rat/mouse simple sthenometer; 200 g scale (O'HARA & CO., LTD.) on the 16th week of age. The survival period of each individual was also analyzed.

### 2. Results

The amount of change in average body weight, food intake, and lower-body skeletal muscle mass of each group after 8-weeks of administration are shown in Table 3.

The amount of change in body weight, food intake, and lower-body skeletal muscle mass after 8-weeks of administration were significantly increased in the human ghrelin group compared with the vehicle group.

**[Table 3]**

| | Vehicle group | Human ghrelin group |
|---|---|---|
| Amount of change in body weight (g) | 0.4 ± 0.4 (15) | 2.6 ± 0.4 (15)** |
| Food intake (g) | 177.0 ± 2.9 (15) | 188.2 ± 3.6 (15)* |
| Lower-body skeletal muscle mass (g) | 4.8 ± 0.3 (15) | 5.7 ± 0.3 (15)* |

| | | |
|---|---|---|
| The numerical values were indicated by mean ± SE (the number of cases) *, **: P < 0.05, 0.01. vs. vehicle group (Student's t test) | | |

The plasma total cholesterol concentration of each group after 8-weeks of administration is shown in Table 4.

No difference was observed between the vehicle group and the human ghrelin group.

**[Table 4]**

| | Vehicle group | Human ghrelin group |
|---|---|---|
| Plasma total cholesterol concentration (mg/dL) | 86.5± 5.6 (15) | 85.8 ± 4.0 (15) |

| | | |
|---|---|---|
| The numerical values were indicated by mean ± SE (the number of cases) | | |

The average forelimb muscle strength of each group after 6-weeks of administration is shown in Table 5.

The human ghrelin group had a significantly stronger forelimb muscle strength than that of the vehicle group, demonstrating that human ghrelin suppressed loss of muscle strength.

**[Table 5]**

| | Vehicle group | Human ghrelin group |
|---|---|---|
| Forelimb muscle strength (N) | 0.74 ± 0.05 (15) | 0.96 ± 0.05 (15)** |

| | | |
|---|---|---|
| The numerical values were indicated by mean ± SE (the number of cases) **: P < 0.01. vs. vehicle group (Student's t test) | | |

Next, the average survival period of each group is shown in Table 6.

The survival period of the human ghrelin group was significantly prolonged compared with the vehicle group and was 13.8% longer on average than the survival days of the vehicle group.

**[Table 6]**

| | Vehicle group | Human ghrelin group |
|---|---|---|
| Survival period (day) | 152 ± 5 (15) | 173 ± 4 (15)** |

| | | |
|---|---|---|
| The numerical values were indicated by mean ± SE (the number of cases) **: P < 0.01. vs. vehicle group (logrank test) | | |

These results demonstrated that human ghrelin, continuously subcutaneously administered from the age of 10 weeks when the SOD1^{G93A} mice already manifested muscle weakness and riluzole was not effective for prolonging their survival periods, increased their body weights and food intakes, suppressed loss of forelimb muscle strength, and prolonged the survival periods, compared with the vehicle group, though having no influence on the plasma total cholesterol concentrations. Thus, human ghrelin was found to suppress the progression of ALS symptoms even under conditions where riluzole, an existing therapeutic agent for ALS, was not effective.

### <Example 4> Effect of human ghrelin on SOD1^{G93A} mice - (2): Motor neuron protective effect by continuous subcutaneous administration

In Example 3, human ghrelin administered to the SOD1^{G93A} mice from the age of 10 weeks was confirmed to suppress loss of forelimb muscle strength and prolong their survival periods. ALS is a disease involving muscle wasting caused by the death of motor neurons, eventually leading to death. In this respect, human ghrelin was examined for its motor neuron protective effect.

### 1. Materials and Methods

In the experiment, 10-week-old SOD1^{G93A} and WT mice were used. The SOD1^{G93A} mice were divided into 2 groups: a vehicle group and a human ghrelin group. Vehicle-administered WT mice were used as a control group. Human ghrelin (50 µg/day, approximately 2 mg/kg/day) was dissolved in a vehicle (saline solution) to prepare a dosing solution. An osmotic pump (ALZET(R) MINI-OSMOTIC PUMP MODEL 1004, DURECT Corporation) filled with the dosing solution or a saline solution was subcutaneously implanted into the back of each mouse for continuous subcutaneous administration. The administration was started from the age of 10 weeks and continued after replacement of the osmotic pump with a fresh one 4 weeks later. Seven weeks after the start of administration, the mice were anatomized, and the T9 regions of their spinal cords were extracted. Nissl-stained sections were prepared, and the number of motor neurons was histologically identified. Three non-adjacent sections were used per individual to measure the number of motor neurons present in the anterior horn. The measurement value of each group was calculated as a relative value when the average number of motor neurons in the control group was defined as 100%.

### 2. Results

The relative number of motor neurons in each group compared with the control group is shown in Table 7.

The number of motor neurons in the vehicle group was as significantly small as approximately 1/2 of that of the control group. On the other hand, the number of motor neurons in the human ghrelin group was significantly larger than that of the vehicle group.

**[Table 7]**

| | Control group | Vehicle group | Human ghrelin group |
|---|---|---|---|
| Mouse genotype | WT | SOD1^{G93A} | SOD1^{G93A} |
| Test substance | Vehicle | Vehicle | Human ghrelin |
| The number of motor neurons (%) | 100 ± 4 (9) | 51 ± 3 (8)** | 84 ± 7 (7) *^{, ##} |

| | | | |
|---|---|---|---|
| The numerical values were indicated by mean ± SE (the number of cases) *, **: P < 0.05, 0.01. vs. control group (Dunnett's multiple comparison test) ^{##}: P < 0.01; vs. vehicle group (Student's t test) | | | |

These results showed that the administration of human ghrelin to the SOD1^{G93A} mice inhibits the decrease in the number of motor neurons, i.e., protects the motor neurons.

### <Example 5> Effect of human ghrelin on SOD1^{G93A} mice - (3): Effects on muscle strength and survival period by repeated subcutaneous administration

In Examples 3 and 4, human ghrelin continuously subcutaneously administered to the SOD1^{G93A} mice from the age of 10 weeks was confirmed to suppress loss of forelimb muscle strength, prolong their survival periods, and protect motor neurons.

In this Example, human ghrelin was examined for its effects on forelimb muscle strengths and survival periods when repeatedly subcutaneously administered to 10-week-old SOD1^{G93A} mice.

### 1. Materials and Methods

In the experiment, 10-week-old SOD1^{G93A} mice were used, which were divided into 2 groups: a vehicle group and a human ghrelin group. Human ghrelin (1 mg/kg) or a vehicle (5% mannitol solution) was subcutaneously administered thereto twice a day from the age of 10 weeks until death. Their body weights and feed weights were measured before the start of administration and after 8-weeks of administration to calculate the amount of change in body weights and the food intakes. The forelimb muscle strengths were measured using a rat/mouse simple sthenometer; 200 g scale (O'HARA & CO., LTD.) before the start of administration and after 8-weeks of administration. The survival period of each individual was also analyzed.

### 2. Results

The amount of change in average body weight and food intake of each group after 8-weeks of administration is shown in Table 8.

The amount of change in body weight and food intake after 8-weeks of administration was significantly increased in the human ghrelin group compared with the vehicle group.

**[Table 8]**

| | Vehicle group | Human ghrelin group |
|---|---|---|
| Amount of change in body weight (g) | 0.1 ± 0.7 (12) | 2.1 ± 0.4 (13)** |
| Food intake (g) | 160.8 ± 3.9 (12) | 176.2 ± 3.5 (13)** |

| | | |
|---|---|---|
| The numerical values were indicated by mean ± SE (the number of cases) ^{**}: P < 0.01. vs. vehicle group (Student's t test) | | |

The average change in forelimb muscle strength of each group from the age of 10 weeks to after 8-weeks of administration is shown in Table 9. The loss of forelimb muscle strength was significantly suppressed in the human ghrelin group compared with the vehicle group.

**[Table 9]**

| | Vehicle group | Human ghrelin group |
|---|---|---|
| Change in forelimb muscle strength (N) | -0.34 ± 0.05 (12) | -0.08 ± 0.07 (13)** |

| | | |
|---|---|---|
| The numerical values were indicated by mean ± SE (the number of cases) **: P < 0.01. vs. vehicle group (Student's t test) | | |

Next, the average survival period of each group is shown in Table 10.

The survival period of the human ghrelin group was significantly prolonged compared with the vehicle group and was 21.8% longer on average than the survival days of the vehicle group.

**[Table 10]**

| | Vehicle group | Human ghrelin group |
|---|---|---|
| Survival period (day) | 147 ± 5 (13) | 179 ± 8 (13)** |

| | | |
|---|---|---|
| The numerical values were indicated by mean ± SE (the number of cases) **: P < 0.01; vs. vehicle group (logrank test) | | |

These results demonstrated that the repeated subcutaneous administration of human ghrelin suppressed the pathological progression of ALS in SOD1^{G93A} mice, as in the continuous subcutaneous administration. This indicated that human ghrelin may suppress the pathological progression of human ALS patients and exhibit a therapeutic effect thereon by repeated subcutaneous administration.

### <Example 6> Effect of human ghrelin on SOD1^{G93A} mice - (4): Effects on body weight, skeletal muscle mass, muscle strength, and the number of motor neurons under restricted feeding

In Examples 3 and 5, human ghrelin continuously subcutaneously administered or repeatedly administered to SOD1^{G93A} mice from the age of 10 weeks was shown to suppress loss of forelimb muscle strength and prolong their survival periods. In Example 4, ghrelin continuously subcutaneously administered to SOD1^{G93A} mice was confirmed to protect motor neurons.

In this Example, SOD1^{G93A} mice were raised under restricted feeding by which the daily food intake of the SOD1^{G93A} mice under voluntary intake conditions was set to approximately 90%. Human ghrelin was continuously subcutaneously administered thereto under conditions where the mice were able to take no more feed, i.e., ghrelin exhibited no orexigenic effect. The administered ghrelin was examined for its effects on body weight, skeletal muscle mass, muscle strength, the number of motor neurons, and mRNA expression of Atrogin-1 and Muscle RING-finger protein-1 (MuRF1), which are both involved in skeletal muscle wasting.

### 1. Materials and Methods

In the experiment, 10-week-old SOD1^{G93A} and WT mice were used. The WT mice were divided into 2 group, one of which was raised under voluntary intake conditions (WT-control group) and the other of which was given 2.8 to 2.9 g/day which corresponded to approximately 90% of the daily food intake under voluntary intake conditions of the SOD1^{G93A} mice (WT-restricted feeding group). The SOD1^{G93A} mice were divided into 2 groups: a vehicle group and a human ghrelin group, both of which were raised under restricted feeding conditions of 2.8 to 2.9 g/day (G93A-vehicle group and G93A-human ghrelin group).

Human ghrelin (50 µg/day, approximately 2 mg/kg/day) was dissolved in a vehicle (saline solution) to prepare a dosing solution. An osmotic pump (ALZET(R) MINI-OSMOTIC PUMP MODEL 1004, DURECT Corporation) filled with the dosing solution or a saline solution was subcutaneously implanted into the back of each mouse for continuous subcutaneous administration. The administration was started from the age of 10 weeks, and the osmotic pump was replaced with a fresh one 4 weeks later.

Their body weights were measured at the start day of administration and after 6 weeks to calculate the amount of change in body weight.

The forelimb muscle strength was measured using a rat/mouse simple sthenometer; 200 g scale (O'HARA & CO., LTD.) 7 weeks after the start of administration.

The lower-body skeletal muscle masses of the mice were measured using X-ray CT (Latheta LCT-200, Hitachi Aloka Medical, Ltd.) before the start of administration (at the age of 10 weeks) and after 7 weeks to determine the amount of change in skeletal muscle mass.

Then, the mice were anatomized, and the T9 regions of their spinal cords were extracted. Nissl-stained sections were prepared, and the number of motor neurons was histologically identified. Three non-adjacent sections were used per individual to measure the number of motor neurons present in the anterior horn. The measurement value of each group was calculated as a relative value when the average number of motor neurons in the WT-control group was defined as 100%.

The gastrocnemial muscles were isolated, and after mRNA extraction, the Atrogin-1 and MuRF1 mRNA expression levels were measured by quantitative PCR. The measurement value of each group was calculated as a relative value when the average mRNA expression level in the WT-control group was defined as 100%.

### 2. Results

All of the mice in the groups raised under restricted feeding ingested the whole portion of feed during the test period. Thus, the food intakes were confirmed to be the same among the WT-restricted feeding group, the G93A-vehicle group, and the G93A-human ghrelin group raised under restricted feeding.

The amount of change in body weight 6 weeks after the start of administration and in lower-body skeletal muscle mass 7 weeks after the start of administration, of the mice in each group, is shown in Table 11.

The body weight of the WT-control group raised under voluntary intake was increased, whereas the restricted feeding decreased body weights both in the WT mice and in the SOD1^{G93A} mice and markedly decreased body weights particularly in the SOD1^{G93A} mice. Decrease in body weight was significantly smaller in the human ghrelin group (G93A-human ghrelin group) than in the SOD1^{G93A} mice given the vehicle (G93A-vehicle group). Similarly, the lower-body skeletal muscle mass was also increased in the WT mice raised under voluntary intake, but decreased in the restricted feeding group. Decrease in skeletal muscle mass was significantly inhibited in the G93A-human ghrelin group compared with the G93A-vehicle group. As shown above, the administration of ghrelin even under restricted feeding conditions where ghrelin exhibited no orexigenic effect suppressed decrease in the body weights or skeletal muscle masses of the SOD1^{G93A} mice.

**[Table 11]**

| | Amount of change in body weight (g) | Amount of change in lower-body skeletal muscle (g) |
|---|---|---|
| WT-control group (9) | 3.5 ± 1.1 | 0.3 ± 0.2 |
| WT-restricted feeding group (8) | -2.5 ± 0.9** | -1.3 ± 0.1** |
| G93A-vehicle group (13) | -4.7 ± 0.6** | -1.6 ± 0.2** |
| G93A-human ghrelin group (15) | -1.4 ± 0.7**^{,##} | -0.9 ± 0.2**^{,#} |

| | | |
|---|---|---|
| ( ): the number of cases, **: P < 0.01; vs. WT-control group (Dunnett's multiple comparison test) ^{#, ##}: P < 0.05, 0.01; vs. G93A-vehicle group (Student's t test) | | |

The mRNA expression levels of Atrogin-1 and MuRF1 in the skeletal muscles 7 weeks after the start of administration are shown in Table 12.

These gene expression levels did not greatly vary between the WT mice raised under restricted feeding and the WT mice raised under voluntary intake, but were significantly elevated in the SOD1^{G93A} mice given the vehicle under restricted feeding (G93A-vehicle group) compared with the WT mice raised under voluntary intake (control group).

On the other hand, the mRNA expressions of Atrogin-1 and MuRF1 were significantly lower in the SOD1^{G93A} mice given human ghrelin under restricted feeding (G93A-ghrelin group) compared with the vehicle group, suggesting that skeletal muscle wasting was suppressed in the human ghrelin group. These results are consistent with the results of Table 11 showing that the decrease in skeletal muscle mass was smaller in the human ghrelin group.

**[Table 12]**

| | Atrogin 1 mRNA expression level (%) | MuRF1 mRNA expression level (%) |
|---|---|---|
| WT-control group (9) | 100 ± 11 | 100 ± 8 |
| WT-restricted feeding group (8) | 113 ± 19 | 142 ± 24 |
| G93A-vehicle group (13) | 528 ± 143** | 613 ± 196* |
| G93A-human ghrelin group (15) | 205 ± 34^{##} | 207 ± 36^{##} |

| | | |
|---|---|---|
| ( ): the number of cases, *^{,}**: P < 0.05, 0.01; vs. WT-control group (Dunnett's multiple comparison test) ^{##}: P < 0.01; vs. G93A-vehicle group (Student's t test) | | |

As shown above, human ghrelin suppressed the skeletal muscle wasting of the SOD1^{G93A} mice even under restricted feeding.

Next, the forelimb muscle strength and the number of motor neurons in the spinal cord of each mouse group under these conditions are shown in Table 13.

The WT mice raised under restricted feeding exhibited forelimb muscle strength or the number of motor neurons equivalent to those of the WT-control group (voluntary intake). The forelimb muscle strength or the number of motor neurons was significantly reduced in the SOD1^{G93A} mice raised under restricted feeding (vehicle) compared with the WT-control group (voluntary intake). This held true for the human ghrelin-administered group. Thus, human ghrelin suppressed neither motor neuron death nor forelimb muscle strength under restricted feeding.

**[Table 13]**

| | The number of motor neurons (%) | Forelimb muscle strength (N) |
|---|---|---|
| WT-control group (9) | 100 ± 11 | 1.02 ± 0.05 |
| WT-restricted feeding group (8) | 93 ± 10 | 0.99 ± 0.07 |
| G93A-vehicle group (13) | 63 ± 4** | 0.74 ± 0.06** |
| G93A-human ghrelin group (15) | 66 ± 5** | 0.77 ± 0.06** |

| | | |
|---|---|---|
| ( ): the number of cases, **: P < 0.01; vs. WT-control group (Dunnett's multiple comparison test) | | |

As seen from these results, ghrelin suppressed the body weight loss or skeletal muscle wasting of the SOD1^{G93A} mice by an action independent from its orexigenic effect even under restricted feeding.

On the other hand, it was shown that, for suppressing motor neuron death or loss of muscle strength, ghrelin must be administered so as to achieve its orexigenic effect, in short, must be administered to an individual whose food intake can be increased by the administration of ghrelin. Specifically, ghrelin was found to improve the systemic energy status by its orexigenic effect, thereby indirectly suppressing motor neuron death and suppressing the pathological progression of ALS.

### <Example 7> Comparison of body weight and forelimb muscle strength between 16-week-old SOD1^{G93A} mice and WT mice

In Examples 3, 4, and 5, human ghrelin administered to SOD1^{G93A} mice from the age of 10 weeks when the mice already manifested forelimb muscle weakness was shown to increase their food intake or body weight, suppress motor neuron death or loss of muscle strength, and prolong the survival period.

In the clinical setting, the treatment of ALS patients was started only when a definite diagnosis of the disease has been made by a clinician after its onset. Since the definite diagnosis of ALS requires half a year to one or more years (Guideline for treatment of ALS, 2002, a guideline for treatment by the Japanese Society of Neurology), the symptoms can be seen to proceed before the start of treatment.

Thus, human ghrelin was examined for its effect when administered to SOD1^{G93A} mice at a more advanced stage in the pathological progression of ALS. In this Example, the body weights and forelimb muscle strengths of 16-week-old SOD1^{G93A} mice were therefore compared with those of WT mice.

### 1. Materials and Methods

In the experiment, 16-week-old WT and SOD1^{G93A} mice were used, and their body weights and forelimb muscle strengths were measured. The forelimb muscle strengths were measured using a rat/mouse simple sthenometer; 200 g scale (O'HARA & CO., LTD.).

### 2. Results

The body weight and forelimb muscle strength of each group are shown in Table 14.

The SOD1^{G93A} mice exhibited an average body weight of at least 2 g smaller and a forelimb muscle strength of approximately 0.5 N lower on average than those of the WT mice. These differences from the values of the WT mice were more marked compared with the mice at the age of 10 weeks (Table 1). Also, the forelimb muscle strength of the 16-week-old SOD1^{G93A} mice was lower than that at the age of 10 weeks (0.91 N) (Table 1).

**[Table 14]**

| | WT mouse | SOD1^{G93A} mouse |
|---|---|---|
| Body weight (g) | 28.0 ± 0.5 (5) | 25.3 ± 0.9 (8)* |
| Forelimb muscle strength | 1.21 ± 0.06(5) | 0.68 ± 0.06 (8)** |

| | | |
|---|---|---|
| The numerical values were indicated by mean ± SE (the number of cases) *^{,} **: P < 0.05, P < 0.01. vs. WT mice (Student's t test) | | |

As shown above, the 16-week-old SOD1^{G93A} mice had a lower forelimb muscle strength compared with the WT mice of the same age or the 10-week-old SOD1^{G93A} mice (Table 1) and were therefore confirmed to be at the more advanced stage in the pathological progression of ALS.

### <Example 8> Effect of human ghrelin on SOD1^{G93A} mice - (5): Effect on survival period by continuous subcutaneous administration from the age of 16 weeks

In this Example, the suppressive effect of human ghrelin on the pathological progression of ALS in 16-week-old SOD1^{G93A} mice that manifested prominent muscle weakness and were at the more advanced stage in the pathological progression of ALS was examined with their survival periods as an index.

### 1. Materials and Methods

In the experiment, 16-week-old SOD1^{G93A} mice were used, which were divided into 2 groups: a vehicle group and a human ghrelin group. Human ghrelin (50 µg/day, approximately 2 mg/kg/day) was dissolved in a vehicle (saline solution) to prepare a dosing solution. An osmotic pump (ALZET(R) MINI-OSMOTIC PUMP MODEL 1004, DURECT Corporation) filled with the dosing solution or a saline solution was subcutaneously implanted into the back of each mouse for continuous subcutaneous administration. The administration was started from the age of 16 weeks and continued to the live individuals with the osmotic pump replaced with a fresh one every 4 weeks. The survival period of each individual was analyzed.

### 2. Results

The average survival period of each group is shown in Table 15.

The survival period of the human ghrelin group was significantly prolonged compared with the vehicle group and was 17.6% longer on average than the survival days of the vehicle group.

**[Table 15]**

| | Vehicle group | Human ghrelin group |
|---|---|---|
| Survival period (day) | 153 ± 7 (9) | 180 ± 11 (9)* |

| | | |
|---|---|---|
| The numerical values were indicated by mean ± SE (the number of cases) *: P < 0.05. vs. vehicle group (logrank test) | | |

As shown above, human ghrelin significantly prolonged the survival periods of the SOD1^{G93A} mice compared with the vehicle group, even when its administration was started from the age of 16 weeks when the SOD1^{G93A} mice manifested prominent muscle weakness and were at the more advanced stage in the pathological progression of ALS.

As shown in Example 2, riluzole, an existing therapeutic agent for ALS, did not prolong survival periods by administration from the age of 10 weeks. These results demonstrated that ghrelin administered to SOD1^{G93A} mice even from the age of 16 weeks exhibits the remarkable effect of significantly prolonging their survival periods, compared with the existing therapeutic agent for ALS.

Thus, ghrelin was found to suppress the pathological progression of ALS remarkably and to have a therapeutic effect.

### <Example 9> Effects of growth hormone secretagogue receptor agonists GHRP-6 and anamorelin on SOD1^{G93A} mice: Effects on survival period by repeated subcutaneous administration

In Example 5, human ghrelin repeatedly subcutaneously administered to SOD1^{G93A} mice from the age of 10 weeks was confirmed to prolong their survival periods.

In this Example, growth hormone secretagogue receptor agonists GHRP-6 and anamorelin were examined for their effects on survival periods when each was repeatedly subcutaneously administered to 10-week-old SOD1^{G93A} mice.

### 1. Materials and Methods

In the experiment, 10-week-old SOD1^{G93A} mice were used, which were divided into 3 groups: a vehicle group, a GHRP-6 group, and an anamorelin group. GHRP-6 (1 mg/kg), anamorelin (1 mg/kg), or a vehicle (5% mannitol solution) was subcutaneously administered thereto twice a day from the age of 10 weeks until death. Their body weight and feed weight were measured before the start of administration and after 5-weeks of administration to calculate the amount of change in body weight and food intake. The survival period of each individual was also analyzed.

### 2. Results

The amount of change in average body weight and food intake of each group after 5-weeks of administration are shown in Table 16.

The amount of change in body weight and food intake after 5-weeks of administration was significantly increased in the GHRP-6 group compared with the vehicle group. The amount of change in body weight after 5-weeks of administration was significantly increased in the anamorelin group compared with the vehicle group, and the food intake also exhibited an increasing tendency in the anamorelin group.

**[Table 16]**

| | Vehicle group | GHRP-6 group | Anamorelin group |
|---|---|---|---|
| Amount of change in body weight (g) | 0.0 ± 0.4 (31) | 1.9 ± 0.2 (30)** | 0.9 ± 0.2 (31)* |
| Food intake (g) | 132.0 ± 2.6 (31) | 142.7 ± 2.1 (30)** | 139.5 ± 2.5 (31)^{†} |

| | | | |
|---|---|---|---|
| The numerical values were indicated by mean ± SE (the number of cases) ⁺, *, **: P < 0.1, P < 0.05, P < 0.01. vs. vehicle group (Dunnett's multiple comparison test) | | | |

Next, the average survival period of each group is shown in Table 17.

The survival periods of the GHRP-6 group and the anamorelin group were significantly prolonged compared with the vehicle group.

**[Table 17]**

| | Vehicle group | GHRP-6 group | Anamorelin group |
|---|---|---|---|
| Survival period (day) | 130 ± 2 (31) | 138 ± 2 (30)* | 136 ± 2 (31)* |

| | | | |
|---|---|---|---|
| The numerical values were indicated by mean ± SE (the number of cases) *: P < 0.05; vs. vehicle group (Wilcoxon test) | | | |

These results demonstrated that the growth hormone secretagogue receptor agonists GHRP-6 and anamorelin repeatedly subcutaneously administered to SOD1^{G93A} mice suppress the pathological progression of ALS, as in human ghrelin.

### Industrial Applicability

A pharmaceutical composition comprising the growth hormone secretagogue receptor agonist or the pharmaceutically acceptable salt thereof can serve as a therapeutic agent for amyotrophic lateral sclerosis in an individual having amyotrophic lateral sclerosis with non-serious dysphagia.

### Free Text for Sequence Listing

SEQ ID NO: 1 - Amino acid sequence of human ghrelin
SEQ ID NO: 2 - Amino acid sequence of human ghrelin (splicing variant, 27 amino acids)

## Claims

1. A therapeutic agent for use in treating amyotrophic lateral sclerosis comprising a growth hormone secretagogue receptor agonist or a pharmaceutically acceptable salt thereof as an active ingredient, wherein the growth hormone secretagogue receptor agonist is ghrelin, GHRP-6, or anamorelin, for administration to an individual having amyotrophic lateral sclerosis with non-serious dysphagia.

2. The therapeutic agent for use according to claim 1, wherein the individual is also unresponsive or insufficiently responsive to an existing therapeutic agent for amyotrophic lateral sclerosis.

3. The therapeutic agent for use according to claim 1 or 2, wherein the therapeutic agent is used in combination with an existing therapeutic agent for amyotrophic lateral sclerosis.

4. The therapeutic agent for use according to claim 2 or 3, wherein the existing therapeutic agent for amyotrophic lateral sclerosis is riluzole.

5. The therapeutic agent for use according to any one of claims 1 to 4, wherein the therapeutic agent is administered by a subcutaneous injection.

6. The therapeutic agent for use according to any one of claims 1 to 5, wherein the ghrelin is a peptide compound comprising the amino acid sequence of SEQ ID NO: 1 in which the 3rd amino acid residue from the amino terminus is a modified amino acid residue with a fatty acid introduced in the side chain of the amino acid residue, or a peptide compound comprising an amino acid sequence of SEQ ID NO: 1 with the deletion, substitution and/or addition of one or several amino acid residues at position 5 to 28 from the amino terminus of SEQ ID NO: 1 in which the 3rd amino acid residue from the amino terminus is a modified amino acid residue with a fatty acid introduced in the side chain of the amino acid residue, and having the activity of elevating an intracellular calcium ion concentration through binding to a growth hormone secretagogue receptor.

7. The therapeutic agent for use according to claim 6, wherein the ghrelin is a peptide compound comprising the amino acid sequence of SEQ ID NO: 1 in which the 3rd amino acid residue from the amino terminus is a modified amino acid residue with a fatty acid introduced at a hydroxy group in the side chain of the amino acid residue.

8. The therapeutic agent for use according to claim 7, wherein the ghrelin is a peptide compound comprising the amino acid sequence of SEQ ID NO: 1 in which the hydroxy group in the side chain of the 3rd amino acid residue from the amino terminus is acylated with a n-octanoyl group.

9. A growth hormone secretagogue receptor agonist or a pharmaceutically acceptable salt thereof for use in treating amyotrophic lateral sclerosis by administration to an individual having amyotrophic lateral sclerosis with non-serious dysphagia, wherein the growth hormone secretagogue receptor agonist is ghrelin, GHRP-6, or anamorelin.

10. The growth hormone secretagogue receptor agonist or the pharmaceutically acceptable salt thereof for use according to claim 9, wherein the individual is also unresponsive or insufficiently responsive to an existing therapeutic agent for amyotrophic lateral sclerosis.

11. The growth hormone secretagogue receptor agonist or the pharmaceutically acceptable salt thereof for use according to claim 9 or 10, wherein in the treatment, the growth hormone secretagogue receptor agonist or the pharmaceutically acceptable salt thereof is used in combination with an existing therapeutic agent for amyotrophic lateral sclerosis.

12. The growth hormone secretagogue receptor agonist or the pharmaceutically acceptable salt thereof for use according to claim 10 or 11, wherein the existing therapeutic agent for amyotrophic lateral sclerosis is riluzole.

13. The growth hormone secretagogue receptor agonist or the pharmaceutically acceptable salt thereof for use according to any one of claims 9 to 12, wherein the administration of the growth hormone secretagogue receptor agonist or a pharmaceutically acceptable salt thereof to the individual is administration in the form of a subcutaneous injection.

14. The growth hormone secretagogue receptor agonist or the pharmaceutically acceptable salt thereof for use according to any one of claims 9 to 13, wherein the ghrelin is a peptide compound comprising the amino acid sequence of SEQ ID NO: 1 in which the 3rd amino acid residue from the amino terminus is a modified amino acid residue with a fatty acid introduced in the side chain of the amino acid residue, or a peptide compound comprising an amino acid sequence of SEQ ID NO: 1 with the deletion, substitution and/or addition of one or several amino acid residues at position 5 to 28 from the amino terminus of SEQ ID NO: 1 in which the 3rd amino acid residue from the amino terminus is a modified amino acid residue with a fatty acid introduced in the side chain of the amino acid residue, and having the activity of elevating an intracellular calcium ion concentration through binding to a growth hormone secretagogue receptor.

15. The growth hormone secretagogue receptor agonist or the pharmaceutically acceptable salt thereof for use according to claim 14, wherein the ghrelin is a peptide compound comprising the amino acid sequence of SEQ ID NO: 1 in which the 3rd amino acid residue from the amino terminus is a modified amino acid residue with a fatty acid introduced at a hydroxy group in the side chain of the amino acid residue.

16. The growth hormone secretagogue receptor agonist or the pharmaceutically acceptable salt thereof for use according to claim 15, wherein the ghrelin is a peptide compound comprising the amino acid sequence of SEQ ID NO: 1 in which the hydroxy group in the side chain of the 3rd amino acid residue from the amino terminus is acylated with a n-octanoyl group.

## Patentansprüche

1. Therapeutisches Mittel zur Verwendung in der Behandlung von amyotropher Lateralsklerose, umfassend einen Wachstumshormon-Sekretagogum-Rezeptoragonist oder ein paharmazeutisch annehmbares Salz davon als einen Wirkstoff, wobei der Wachstumshormon-Sekretagogum-Rezeptoragonist Grehlin, GHRP-6, oder Anamorelin ist, für die Verabreichung an ein Individuum, das amyotrophe Lateralsklerose im nichtschweren Fall von Dysphagie aufweist.

2. Therapeutisches Mittel zur Verwendung nach Anspruch 1, wobei das Individuum auch auf ein bestehendes therapeutisches Mittel für amyotrophe Lateralsklerose nicht anspricht oder nicht ausreichend anspricht.

3. Therapeutisches Mittel nach Anspruch 1 oder 2, wobei das therapeutische Mittel in Kombination mit einem bestehenden therapeutischen Mittel für amyotrophe Lateralsklerose verwendet wird.

4. Therapeutisches Mittel nach Anspruch 2 oder 3, wobei das bestehende therapeutische Mittel für amyotrophe Lateralsklerose Riluzol ist.

5. Therapeutisches Mittel nach einem der Ansprüche 1 bis 4, wobei das therapeutische Mittel mit Hilfe einer subkutanen Injektion verabreicht wird.

6. Therapeutisches Mittel nach einem der Ansprüche 1 bis 5, wobei das Ghrelin eine Peptidverbindung ist, die die Aminosäuresequenz von SEQ ID NO: 1 umfasst, in der der 3. Aminosäurerest von dem Aminoterminus ein modifizierter Aminosäurerest mit einer in die Seitenkette des Aminosäurerests eingeführten Fettsäure ist, oder eine Peptidverbindung, die eine Aminosäuresequenz von SEQ ID NO: 1 mit der Deletion, Substitution und/oder Addition von einem oder mehreren Aminosäureresten in Position 5 bis 28 von dem Aminoterminus von SEQ ID NO: 1 umfasst, in dem der 3. Aminosäurerest von dem Aminoterminus ein modifizierter Aminosäurerest mit einer in die Seitenkette des Aminosäurerests eingeführten Fettsäure ist, und die die Aktivität aufweist, eine intrazelluläre Konzentration von Calciumionen durch Binden an ein Wachstumshormon-Sekretagogum-Rezeptor zu erhöhen.

7. Therapeutisches Mittel zur Verwendung nach Anspruch 6, wobei das Ghrelin eine Peptidverbindung ist, die die Aminosäuresequenz von SEQ ID NO: 1 umfasst, in der der 3. Aminosäurerest von dem Aminoterminus ein modifizierter Aminosäurerest mit einer an einer Hydroxy-Gruppe in die Seitenkette des Aminosäurerests eingeführten Fettsäure ist.

8. Therapeutisches Mittel zur Verwendung nach Anspruch 7, wobei das Ghrelin eine Peptidverbindung ist, die die Aminosäuresequenz von SEQ ID NO: 1 umfasst, in der die Hydroxy-Gruppe in der Seitenkette des 3. Aminosäurerests von dem Aminoterminus mit einer n-Octanoyl-Gruppe acyliert ist.

9. Wachstumshormon-Sekretagogum-Rezeptoragonist oder pharmazeutisch annehmbares Salz davon zur Verwendung in der Behandlung von amyotropher Lateralsklerose durch Verabreichung an ein Individuum, das amyotrophe Lateralsklerose im nichtschweren Fall von Dysphagie aufweist, worin der Wachstumshormon-Sekretagogum-Rezeptoragonist Grehlin, GHRP-6, oder Anamorelin ist.

10. Wachstumshormon-Sekretagogum-Rezeptoragonist oder ein pharmazeutisch annehmbares Salz davon zur Verwendung nach Anspruch 9, wobei das Individuum auch auf ein bestehendes therapeutisches Mittel für amyotrophe Lateralsklerose nicht anspricht oder nicht ausreichend anspricht.

11. Wachstumshormon-Sekretagogum-Rezeptoragonist oder pharmazeutisch annehmbares Salz davon zur Verwendung nach Anspruch 9 oder 10, wobei in der Behandlung, der Wachstumshormon-Sekretagogum-Rezeptoragonist oder das pharmazeutisch annehmbare Salz davon in Kombination mit einem bestehenden therapeutischen Mittel für amyotrophe Lateralsklerose verwendet wird.

12. Wachstumshormon-Sekretagogum-Rezeptoragonist oder pharmazeutisch annehmbares Salz davon zur Verwendung nach Anspruch 10 oder 11, wobei das bestehende therapeutische Mittel für amyotrophe Lateralsklerose Riluzol ist.

13. Wachstumshormon-Sekretagogum-Rezeptoragonist oder pharmazeutisch annehmbares Salz davon zur Verwendung nach einem der Ansprüche 9 bis 12, wobei die Verabreichung des Wachstumshormon-Sekretagogum-Rezeptoragonisten oder eines pharmazeutisch annehmbaren Salzes davon an das Individuum eine Verabreichung in der Form einer subkutanen Injektion ist.

14. Wachstumshormon-Sekretagogum-Rezeptoragonist oder pharmazeutisch annehmbares Salz davon zur Verwendung nach einem der Ansprüche 9 bis 13, wobei das Ghrelin eine Peptidverbindung ist, die die Aminosäuresequenz von SEQ ID NO: 1 umfasst, in der der 3. Aminosäurerest von dem Aminoterminus ein modifizierter Aminosäurerest mit einer in die Seitenkette des Aminosäurerests eingeführten Fettsäure ist, oder eine Peptidverbindung, die eine Aminosäuresequenz von SEQ ID NO: 1 mit der Deletion, Substitution und/oder Addition von einem oder mehreren Aminosäureresten in Position 5 bis 28 von dem Aminoterminus von SEQ ID NO: 1 umfasst, in dem der 3. Aminosäurerest von dem Aminoterminus ein modifizierter Aminosäurerest mit einer in die Seitenkette des Aminosäurerests eingeführten Fettsäure ist, und mit einer Aktivität des Erhöhens einer intrazellulären Konzentration von Calciumionen durch Binden an ein Wachstumshormon-Sekretagogum-Rezeptor.

15. Wachstumshormon-Sekretagogum-Rezeptoragonist oder pharmazeutisch annehmbares Salz davon zur Verwendung nach Anspruch 14, wobei das Ghrelin eine Peptidverbindung ist, die die Aminosäuresequenz von SEQ ID NO: 1 umfasst, in der der 3. Aminosäurerest von dem Aminoterminus ein modifizierter Aminosäurerest mit einer an einer Hydroxy-Gruppe in die Seitenkette des Aminosäurerests eingeführten Fettsäure ist.

16. Wachstumshormon-Sekretagogum-Rezeptoragonist oder pharmazeutisch annehmbares Salz davon zur Verwendung nach Anspruch 15, wobei das Ghrelin eine Peptidverbindung ist, die die Aminosäuresequenz von SEQ ID NO: 1 umfasst, in der die Hydroxy-Gruppe in der Seitenkette des 3. Aminosäurerests von dem Aminoterminus mit einer n-Octanoyl-Gruppe acyliert ist.

## Revendications

1. Agent thérapeutique pour l'utilisation dans le traitement d'une sclérose latérale amyotrophique, comprenant un agoniste des récepteurs des sécrétagogues de l'hormone de croissance ou un sel pharmaceutiquement acceptable de celui-ci en tant qu'ingrédient actif, dans lequel l'agoniste du récepteur des sécrétagogues est la ghréline, GHRP-6, ou l'anamoréline, pour l'administration à une personne ayant une sclérose latérale amyotrophique avec dysphagie non grave.

2. Agent thérapeutique pour l'utilisation selon la revendication 1, dans lequel la personne est aussi insensible ou insuffisamment sensible à un agent thérapeutique existant pour la sclérose latérale amyotrophique.

3. Agent thérapeutique pour l'utilisation selon la revendication 1 ou 2, lequel agent thérapeutique est utilisé en combinaison avec un agent thérapeutique existant pour la sclérose latérale amyotrophique.

4. Agent thérapeutique pour l'utilisation selon la revendication 2 ou 3, dans lequel l'agent thérapeutique existant pour la sclérose latérale amyotrophique est le riluzole.

5. Agent thérapeutique pour l'utilisation selon l'une quelconque des revendications 1 à 4, lequel agent thérapeutique est administré par une injection sous-cutanée.

6. Agent thérapeutique pour l'utilisation selon l'une quelconque des revendications 1 à 5, dans lequel la ghréline est un composé peptidique comprenant la séquence d'acides aminés de SEQ ID NO: 1 dans laquelle le 3^{eme} reste d'acide aminé à partir de l'extrémité amino est un reste d'acide aminé modifié avec un acide gras introduit dans la chaîne latérale du reste d'acide aminé, ou un composé peptidique comprenant une séquence d'acides aminés de SEQ ID NO: 1 avec la délétion, substitution et/ou addition d'un ou plusieurs restes d'acides aminés aux positions 5 à 28 à partir de l'extrémité amino de la SEQ ID NO: 1 dans laquelle le 3^{eme} reste d'acide aminé à partir de l'extrémité amino est un reste d'acide aminé modifié avec un acide gras introduit dans la chaîne latérale du reste d'acide aminé, et a l'activité d'élévation d'une concentration d'ions calcium intracellulaire par liaison à un récepteur sécrétagogue d'hormone de croissance.

7. Agent thérapeutique pour l'utilisation selon la revendication 6, dans lequel la ghréline est un composé peptidique comprenant la séquence d'acides aminés de SEQ ID NO: 1 dans laquelle le 3^{eme} reste d'acide aminé à partir de l'extrémité amino est un reste d'acide aminé modifié avec un acide gras introduit à un groupe hydroxy dans la chaîne latérale du reste d'acide aminé.

8. Agent thérapeutique pour l'utilisation selon la revendication 7, dans lequel la ghréline est un composé peptidique comprenant la séquence d'acides aminés de SEQ ID NO: 1 dans laquelle le groupe hydroxy dans la chaîne latérale du 3^{eme} reste d'acide aminé à partir de l'extrémité amino est acylé avec un groupe n-octanoyle.

9. Agoniste du récepteur des sécrétagogues de l'hormone de croissance ou un sel pharmaceutiquement acceptable de celui-ci pour l'utilisation dans le traitement d'une sclérose latérale amyotrophique par administration à une personne ayant une sclérose latérale amyotrophique avec dysphagie non grave, dans lequel l'agoniste du récepteur des sécrétagogues de l'hormone de croissance est la ghréline, GHRP-6, ou l'anamoréline,

10. Agoniste du récepteur des sécrétagogues de l'hormone de croissance ou sel pharmaceutiquement acceptable de celui-ci pour l'utilisation selon la revendication 9, dans laquelle la personne est aussi insensible ou insuffisamment sensible à un agent thérapeutique existant pour la sclérose latérale amyotrophique.

11. Agoniste du récepteur des sécrétagogues de l'hormone de croissance ou sel pharmaceutiquement acceptable de celui-ci pour l'utilisation selon la revendication 9 ou 10, dans laquelle, lors du traitement, l'agoniste du récepteur des sécrétagogues de l'hormone de croissance ou le sel pharmaceutiquement acceptable de celui-ci est utilisé en combinaison avec un agent thérapeutique existant pour la sclérose latérale amyotrophique.

12. Agoniste du récepteur des sécrétagogues de l'hormone de croissance ou sel pharmaceutiquement acceptable de celui-ci pour l'utilisation selon la revendication 10 ou 11, dans laquelle l'agent thérapeutique existant pour la sclérose latérale amyotrophique est le riluzole.

13. Agoniste du récepteur des sécrétagogues de l'hormone de croissance ou sel pharmaceutiquement acceptable de celui-ci pour l'utilisation selon l'une quelconque des revendications 9 à 12, dans laquelle l'administration de l'agoniste du récepteur des sécrétagogues de l'hormone de croissance ou d'un sel pharmaceutiquement acceptable de celui-ci à la personne est une administration sous la forme d'une injection sous-cutanée.

14. Agoniste du récepteur des sécrétagogues de l'hormone de croissance ou un sel pharmaceutiquement acceptable de celui-ci pour l'utilisation selon l'une quelconque des revendications 9 à 13, dans lequel la ghréline est un composé peptidique comprenant la séquence d'acides aminés de SEQ ID NO: 1 dans laquelle le 3^{eme} reste d'acide aminé à partir de l'extrémité amino est un reste d'acide aminé modifié avec un acide gras introduit dans la chaîne latérale du reste d'acide aminé, ou un composé peptidique comprenant une séquence d'acides aminés de SEQ ID NO: 1 avec la délétion, substitution et/ou addition d'un ou plusieurs restes d'acides aminés aux positions 5 à 28 à partir de l'extrémité amino de la SEQ ID NO: 1 dans laquelle le 3^{eme} reste d'acide aminé à partir de l'extrémité amino est un reste d'acide aminé modifié avec un acide gras introduit dans la chaîne latérale du reste d'acide aminé, et a l'activité d'élévation d'une concentration d'ions calcium intracellulaire par liaison à un récepteur sécrétagogue d'hormone de croissance.

15. Agoniste du récepteur des sécrétagogues de l'hormone de croissance ou sel pharmaceutiquement acceptable de celui-ci pour l'utilisation selon la revendication 14, dans lequel la ghréline est un composé peptidique comprenant la séquence d'acides aminés de SEQ ID NO: 1 dans laquelle le 3^{eme} reste d'acide aminé à partir de l'extrémité amino est un reste d'acide aminé modifié avec un acide gras introduit à un groupe hydroxy dans la chaîne latérale du reste d'acide aminé.

16. Agoniste du récepteur des sécrétagogues de l'hormone de croissance ou sel pharmaceutiquement acceptable de celui-ci pour l'utilisation selon la revendication 15, dans lequel la ghréline est un composé peptidique comprenant la séquence d'acides aminés de SEQ ID NO: 1 dans laquelle le groupe hydroxy dans la chaîne latérale du 3^{eme} reste d'acide aminé à partir de l'extrémité amino est acylé avec un groupe n-octanoyle.
